(19) Europäisches Patentamt
European Patent Office
Office européen des brevets

(11) Veröffentlichungsnummer: **0 400 319 A1**

(12) **EUROPÄISCHE PATENTANMELDUNG**

(21) Anmeldenummer: 90107538.2

(51) Int. Cl.5: **C07D 217/14, A61K 31/47**

(22) Anmeldetag: 20.04.90

(30) Priorität: 24.04.89 BG 88220/89

(43) Veröffentlichungstag der Anmeldung:
05.12.90 Patentblatt 90/49

(84) Benannte Vertragsstaaten:
BE CH DE DK FR GB IT LI NL SE

(71) Anmelder: N I S PRI VCHTI
Boul. Kl. Ohridski 8
Sofia(BG)

(72) Erfinder: Mondeshka, Donka M.
ul. Rakovski 125
Sofia(BG)
Erfinder: Ivanova, Nedyalka St.
ul. Tr. Kostov 11
Sofia(BG)
Erfinder: Ivanov, Chavdar B.
Komplex Mladost 1, Bl. 46-4
Sofia(BG)
Erfinder: Angelova, Ivanka G.
ul. G. Genov 19
Sofia(BG)
Erfinder: Boyadjiev, Stefan Em.
ul. Sheinovo 45
Sofia(BG)
Erfinder: Tancheva, Chonka N.
Komplex Mladost 1, Bl. 80-2
Sofia(BG)
Erfinder: Tersiiska, Spaska An.
ul. V. Dimitrov, Bl. 244-2
Sofia(BG)
Erfinder: Tosheva, Mariana R.
ul. Br. Zlatarski 57
Ihtiman(BG)
Erfinder: Parashikov, Vladimir At.
Komplex Ovcha Kupel-2, Bl. 29A-A
Sofia(BG)
Erfinder: Paskov, Vladimir D.
ul. Kiril i Metodi 43
Sofia(BG)
Erfinder: Velkov, Vesselin An.
ul. Bogdan, Bl. 34
Sofia(BG)

(74) Vertreter: von Füner, Alexander, Dr. et al
Patentanwälte v. Füner, Ebbinghaus, Finck
Mariahilfplatz 2 & 3
D-8000 München 90(DE)

(54) **4-(4'-Halophenyl)-2-methyl-1,2,3,4-tetrahydroisochinoline und Verfahren zu ihrer Herstellung.**

(57) 4-(4'-Halophenyl)-2-methyl-1,2,3,4-tetrahydroisochinolin, Razemate oder optisch aktive Isomere der Formel 1

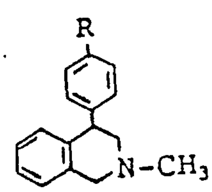

wobei R Brom oder Fluor bedeutet und ihre physiologisch verträgliche Sälze, sowie ein Verfahren zu ihrer Herstellung.

Die erfindungsgemäßen Verbindungen haben eine hohe inhibierende Wirkung auf das uptake des Dopamins (DA), des Noradrenalins (NA) und des Serotonins (5-HT) und weisen eine hohe Antiulkusaktivität auf.

EP 0 400 319 A1

# 4-(4'-HALOPHENYL)-2-METHYL-1,2,3,4-TETRAHYDROISOCHINOLINE UND VERFAHREN ZU IHRER HERSTELLUNG

Die Erfindung betrifft 4-(4'-Halophenyl)-2-methyl-1,2,3,4-tetrahydroisochinoline, Razemate oder optisch aktive Isomere sowie ein Verfahren zu ihrer Herstellung.

Bekannt sind razemische oder optisch aktive 4-Phenyl-8-amino-2-methyl-1,2,3,4-tetrahydroisochinoline mit inhibierender Wirkung des reuptake's von Dopamin (DA), Noradrenalin (NA) und Serotonin (5-HT) aus Synaptosomen der Hirnrinde von Ratten (R. Kunstmen, H. Gerhards, H. Kruse, M. Leven. E. Paulus, U. Schacht, K. Schmitt, J.Med.Chem. 1987, 30,798-804).

Weiterhin ist es bekannt, daß das razemische 4-Phenyl-8-amino-2-methyl-1,2.3,4-tetrahydroisochinolin eine mäßig inhibierende Wirkung auf durch Streß verursachte Magengeschwüre bei Ratten ausübt (M. Bickel, Arzneim.Forsch., Drug Res., 30 (1), Nr. I, 1980, 69).

Aufgabe der Erfindung ist es, neue 4-(4'-Halophenyl)-2-methyl-1,2,3,4-tetrahydroisochinoline der Formel 1

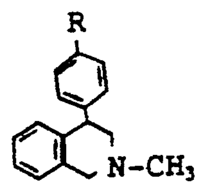

(1)

(1a = Br)

(1b = F)

wobei R Brom oder Fluor bedeuten, sowie deren physiologisch verträgliche Salze mit einer hohen inhibierenden Wirkung auf das reuptake des Dopamins, Noradrenalins oder Serotonins und einer hohen Antiulkusaktivität zu synthetisieren.

Das Wesen der Erfindung besteht in der Synthese der razemischen Verbindungen der allgemeinen Formel 1 nach folgendem Dreistufenschema:

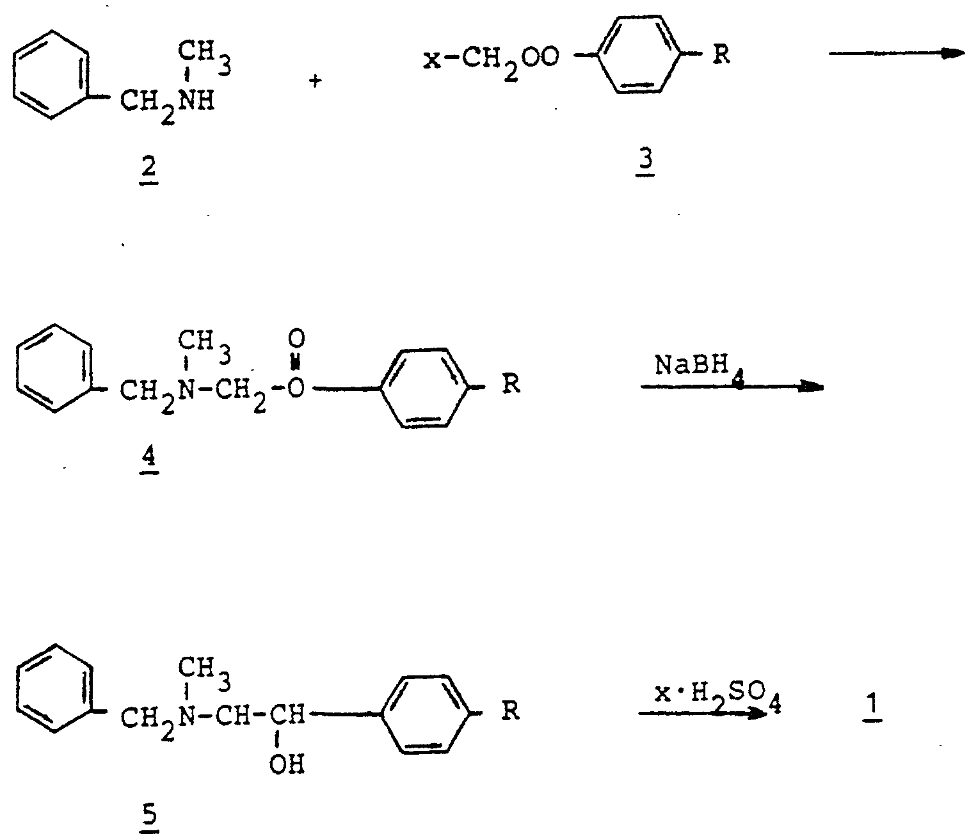


2 3 4 5 1

a) Alkylierung der freien Base Benzylmethylamin 2, in doppelt molarem Überschuß, mit α-halogenarylaliphatischen Ketonen 3 in kochendem Benzol oder Aceton bis zum Erhalt von 2-(N-Methyl-N-benzylamino)-4-haloacetophenonen, wobei R die oben erwähnte Bezeichnung hat. Die Ketone 4 werden als Hydrochloride nach Behandlung der rohen Basen mit trockenem Diethylether, gesättigt mit Chlorwasserstoff, erhalten.

b) Reduktion der Ketone 4 zu N-Benzyl-2-(methylamino)-1-(4-halophenyl)-1-ethanolen 5 unter Verwendung von dreifach molarem Überschuß an Natriumborhydrid. Die Reaktion verläuft in einem Medium von niedrigen Alkoholen in einem Temperaturbereich von 10 bis 80° C. Die erhaltenen Carbinole 5 werden als gut kristallisierende freie Basen in dünnschichtchromatographisch reiner Form isoliert und der weiteren Cyclodehydratisierung unterzogen.

c) Die Cyclodehydratisierung der Carbinole 5 zu 4-Aryltetrahydroisochinolinen 1 erfolgt in Dichlormethan oder Dichlorethan in Anwesenheit von konz. Schwefelsäure im Temperaturbereich von 0 bis 30° C, vorzugsweise 10 bis 15° C, während 1 bis 4 h, vorzugsweise 1 bis 2 h.

Die rohen Basen werden aus dem Reaktionsgemisch isoloert, indem es auf Eiswasser gegossen wird, die Lösung dann mit 25%igem Ammoniak neutralisiert und mit einem geeigneten organischen Lösungsmittel extrahiert wird.

Die Hydrochloride von 1 werden aus den rohen Basen durch Auflösen der letzteren in absolutem Alkohol und Behandlung der erhaltenen Lösungen mit an Chlorwasserstoff gesättigten Diethylether erhalten.

Die Struktur der Verbindungen der allgemeinen Formel 1 wird mit Hilfe von IR, $^1$H-NMR und Massenspektren sowie Elementaranalyse ermittelt.

Die optisch aktiven Formen von 1a (R = Br) erhält man, indem die (±)1a freie Base mit (+)-0,0-Dibenzoyl-D-weinsäure oder (-)-0,0-Dibenzoyl-L-weinsäure in 96% Ethanol behandelt wird.

Die Verbindung der allgemeinen Formel 1 wurden auf den Einfluß auf das reuptake des Dopamins (DA), des Noradrenalins (NA) und des Serotonins (5-HT) aus Synaptosomen der Hirnrinde von Ratten untersucht. Diese biochemischen Tests gelten als Grundkriterien für die Anwesenheit antidepressiver Aktivität. Aus den Ergebnissen (Tabelle 1) ist ersichtlich, daß die razemischen 1a- und 1b-Verbindungen nicht nur eine hohe inhibierende Wirkung in bezug auf den uptake von DA und NA aufweisen, sondern auch starke Inhibitoren des uptake's von 5-HT darstellen, im Vergleich zum bekannten Antidepressivum Nomifensin, einem schwachen Serotonininhibitor.

Untersucht wurde auch die Antiulkusaktivität der razemischen 1a- und 1b-Verbindungen auf ein experimentelles Modell von Wasser-Immersionsstreß verursachten Magengeschwüren bei Ratten (Tabelle 2). Das Vergleichspräparat war der $H_2$-Antagonist Ranitidin.

Die Ergebnisse dieser Untersuchungen zeigen, daß die oral verabreichte Verbindung 1a eine hohe Antiulkusaktivität noch bei Dosen von 0,050 mg/kg besitzt, mit einem Prozentsatz der Unterdrückung des Magengeschwürindex von 20. Die durchschnittliche effektive Dosis liegt bei 0,500 mg/kg, bei der der Inhibierungsgrad 52% beträgt. Bei einer Erhöhung der Dosis steigt der Inhibierungsgrad an und erreicht ein Maximum bei einer Dosis von 3 mg/kg.

Im Vergleich zu Ranitidin erwies es sich, daß 1a eine mehrfach höhere Antiulkuswirkung aufweist. So z.B. beträgt der Inhibierungsgrad des Magengeschwürindex bei einer Dosis von 0,100 mg/kg 48,9% für 1a, bei einer fünfmal höheren Dosis von Ranitidin (0,500 mg/kg) dagegen nur 27%. Bei einer Dosis von 0,500 mg/kg von 1a beträgt der Inhibierungsgrad 52%. Dieser Prozentsatz wird mit einer Dosis von 10 mg/kg Ranitidin erreicht.

Dieses Ergebnis zeigt eine bei 1a 20 Mal höhere Antiulkuswirkung im Vergleich zum Präparat Ranitidin. Ähnliche Ergebnisse wurden auch mit 1b erreicht.

## Beispiel 1

Herstellung von 2-(N-Methyl-N-benzylamino)-4-bromacetophenon 4a (R = Br)

Zu einer Lösung von 6,05 g (50 mmol) freier Base Benzylmethylamin in 15 ml Aceton wird bei 35 bis 40 °C eine Lösung von 6,95 g (25 mmol) ω-Brom-4-bromacetophenon in 25 ml Aceton gegeben. Das Reaktionsgemisch kocht 8 h, worauf man es im Kühlschrank stehenläßt. Das auskristallisierte Hydrochlorid des Ausgangsbenzylmethylamins wird abfiltriert, das Filtrat i. vac. bis zum Erhalt eines öligen Rückstands eingedampft und in 5 ml absolutem Alkohol gelöst. Zu der Lösung wird mit Chlorwasserstoff gesättigter, trockener Diethylether gegeben. Zur vollständigen Abscheidung des Hydrochlorids von 4a werden zusätzlich 50 ml Diethylether zugefügt und aus Ethanol umkristallisiert. Die Ausbeute beträgt 6.09 g (70%) 4a-Hydrochlorid, Schmelzp. 133-138 °C.
IR-Spektrum (KBr): $cm^{-1}$ 1690 (CO).

| Elementaranalyse: $C_{15}H_{18}NOClBr$ | |
|---|---|
| berechnet in %: | C 54,08, N 3,93, Cl 9,96, Br 22,46 |
| gefunden in %: | C 54,20, H 4,98, N 4,01, Cl 9,80, Br 22,30 |

Analog zu diesem Verfahren wurde 2-(N-Methyl-N-benzylamino)-4-fluoracetophenon 4b (R = F) mit einer Ausbeute von 79% erhalten.

| Elementaranalyse: $C_{15}H_{18}NOClF$ | |
|---|---|
| berechnet in %: | C 65,19, H 6,16, N 4,75, Cl 12,03 |
| gefunden in %: | C 65,22, H 6,10, N 4,80, Cl 12,10 |

## Beispiel 2

Herstellung von N-Benzyl-2-(methylamino)-1-(4-bromphenyl)-1-ethanol 5 (R = Br).

In 120 ml Methanol wurden 12,30 g (37 mmol) 2-(N-Methyl-N-benzylamino)-4-bromacetophenon Hydrochlorid gelöst und unter Rühren bei ca. 10 °C 5,32 g (139 mmol) Natriumborhydrid während 30 Minuten zugegeben. Man rührt bei Zimmertemperatur weitere 30 min, wonach das Reaktionsgemisch im Laufe von

6 h kocht. Das Lösungsmittel wird i.vac. eingedampft und zum Rückstand 150 ml Wasser gegeben. Das entstandene Öl wird viermal mit Dichlormethan extrahiert, der Extrakt mit Natriumsulfat getrocknet und das Lösungsmittel i.vac. entfernt. Ausbeute 8,5 g (82%) von DC reiner Base 5a, Schmelzp. 60-61,5° C. IR-Spektrum (KBr) $cm^{-1}$: 3200 (OH).

Analog zu diesem Verfahren wurde N-Benzyl-2-(methylamino)-1-(4-fluorphenyl)-ethanol 5b (R = F) erhalten. Die Ausbeute beträgt 94%.

| Elementaranalyse (5a): $C_{16}H_{18}NOBr$ | |
|---|---|
| berechnet in %:<br>gefunden in %: | C 60,02, H 5,66, N 4,37, Br 24,95<br>C 60,10, H 5,40, N 4,20, Br 24,85 |

| Elementaranalyse (5b): $C_{16}H_{18}NOF$ | |
|---|---|
| berechnet in %:<br>gefunden in %: | C 74,19, H 6,99, N 5,40<br>C 74,30, H 7,10, N 5,20 |

Beispiel 3

Herstellung von 4-(4′-Bromphenyl)-2-methyl-1,2,3,4-tetrahydroisochinolin Hydrochlorid 1a (R = Br)

Zu 11,7 ml auf 5° C abgekühlte konz. Schwefelsäure wird im Laufe von 45 min unter intensivem Rühren eine Lösung von 2,98 g (9,3 mmol) N-Benzyl-2-methylamino-1-(4′bromphenyl)-1-ethanol in 25 ml trockenem Dichlormethan zugetropft. Das Reaktionsgemisch wird bei derselben Temperatur noch 1 h gerührt und dann auf 50 g feingehacktes Eis und 50 ml Wasser gegossen. Die organische Schicht wird abgetrennt, die wäßrige mit 25%igem Ammoniak alkalisiert und das entstandene Öl mit Dichlormethan extrahiert. Der Extrakt wird mit Natriumsulfat getrocknet, das Lösungsmittel i.vac. abdestilliert und die freie ölige Base 1a in einer minimalen Methanolmenge gelöst. Nach Zugabe von trockenem Diethylether, der mit Chlorwasserstoff gesättigt ist, entsteht das Hydrochlorid, das aus Methanol-Diethylether umkristallisiert wird. Die Ausbeute beträgt 2,57 g (82%) 1a.

| Elementaranalyse $C_{16}H_{17}NClBr$ | |
|---|---|
| berechnet in %:<br>gefunden in %: | C 56,74, H 5,60, N 4,13<br>C 56,64, H 5,20, N 4,22 |

$^1H$-NMR ppm (DMSO-$d_6$): 2,56 (s, 3H, -N-$CH_3$), 3,32-3,76 (m, 2H) und 4,53 (m, 1H, -$CH_2$(3)-CH(4)), 4,24 und 4,36 (m, 2H, J = 8,5 Hz, -1-$CH_2$), 6,65-7,40 (2 m, 8H Ar, -O-$C_6H_4$ und p-$C_6H_4$).
MS (70 eV): m/z = 303/301 (1:1) für $^{81}Br/^{79}Br$ (Base).

Analog zu diesem Verfahren wurde 4-(4′-fluorphenyl)-2-methyl-1,2,3,4-tetrahydroisochinolin Hydrochlorid 1b (R = F) mit einer Ausbeute von 86% und einem Schmelzp. von 136-140° C hergestellt.

| Elementaranalyse: $C_{16}H_{17}NClF$ | |
|---|---|
| berechnet in %:<br>gefunden in %: | C 69,19, H 6,17, N 5,04<br>C 69,30, H 6,35, N 5,15 |

$^1H$-NMR ppm (DMSO-$d_6$): 2,52 (s, 3H, -N-$CH_3$), 3,2 0-3,60 (m, 2H) und 4,40 (m, 1H -$CH_2$(3)-CH(1)), 4,12

und 4,26 (m, 2H, J = 10 Hz, 1-$CH_2$), 6,78-7,36 (2 m, 8H Ar, -o-$C_6H_4$ und p-$C_6H_5$). MS (70 ev): m/z = 241 ($M^+$).

Beispiel 4

Herstellung von optisch aktiven Formen von 4-(4′-Bromphenyl)-2-methyl-1,2,3,4-tetrahydroisochinolin 1a

9 g (0,03 mol) razemische freie Base 1a wird unter Kochen in 90 ml Ethanol gelöst und der Lösung 10,75 g (0,03 mol) (+)-0,0-Dibenzoyl-D-weinsäure in 90 ml Ethanol zugegeben. Nach 24stündigem Stehenlassen bei 8 bis 10 °C kristallisieren 9,5 g (96,4% der theoretischen Ausbeute) Dibenzoyl-D-(+)-Tartrat von 1a, Schmelzp. 115-119 °C und $[\alpha]_D^{20}$ = + 68,08 (c = 0,25, $CH_3OH$), $[\alpha]_D^{20}$ der freien Base beträgt + 31,32 (c = 0,25, $CHCl_3$). Nach einmaliger Umkristallisierung des rohen Tartrats aus Ethanol entstehen 8,2 g (87% der theoretischen Ausbeute) 1a Tartrat mit $[\alpha]_D^{20}$ = + 82,38, der Schmelzp. beträgt 118-121 °C. Die freie Base erhält man nach Behandlung des Tartrats mit wäßriger Ammoniaklösung und darauffolgender Extraktion mit Chloroform. (+) 1a, $[\alpha]_D^{20}$ = + 34,72 (c = 0,25, $CHCl_3$).

Die vereinten Mutterlaugen des rohen Tartrats sowie jene aus der ersten Umkristallisierung werden i.vac. bis zur Trockne eingedampft. Der Rückstand wird in Wasser gelöst, mit Ammoniak alkalisiert und das entstandene Öl mit Chloroform extrahiert. Der Extrakt wird mit Natriumsulfat getrocknet, das Lösungsmittel i.vac, eingedampft und die entstandene rohe Base (-) 1a (7,5 g) in heißem Ethanol (37,5 ml) gelöst. Zu der Lösung werden 8,96 g (0,025 mol) (-)-0,0-Dibenzoyl-L-weinsäure zugefügt. Nach Stehenlassen bei 8 bis 10 °C erhält man 5,2 g rohes Tartrat (-) 1a, $[\alpha]_D^{20}$ = -68,08 (c = 0,25, $CH_3OH$). Nach einmaliger Umkristallisierung des rohen Tartrats aus Ethanol erhält man 4,8 g (92% der theoretischen Ausbeute) (-) 1a Tartrat, $[\alpha]_D^{20}$ = -74,89 (c = 0,25, $CH_3OH$) und Schmelzp. 120-124 °C (-) 1a freie Base, $[\alpha]_D^{20}$ = -30,64 (c = 0,25, $CHCl_3$).

Beispiel 5

Bestimmung der inhibierenden Wirkung von 4-(4′-Halophenyl)-2-methyl-1,2,3,4-tetrahydroisochinolin 1a und 1b, uptake von DA, DN und 5-HT aus Synaptosomen, isoliert aus der Hirnrinde von Ratten

Für jeden Versuch wurden männliche weiße Ratten der Rasse Wistar verwendet, die dekapitiert wurden. Das Gehirn wurde herausgeschnitten, der Hypothalamus, Corpus stritum und die spezifischen Gehirnteile isoliert, gewogen und in 10 ml 0,32 M Saccharose homogenisiert. Die an Synaptosomen $P_2$ angereicherten Fraktionen für jeden Gehirnteil werden nach dem Verfahren (E.C. Gray, V.R. Whittaker, J. Anatomy, 1962, 96, 79) bearbeitet und einmal mit derselben Lösung gewaschen. Jede Fraktion wird bei 11000 g 20 min zentrifugiert und in 20-50 Volumen des Puffers bei einem pH von 7,4 und 37 °C resuspendiert. Puffer: 134 mM NaNaCl, 4,1 mM KCl, 1,1 mM $KH_2PO_4$, 1,2 mM $MgCl_2$, 5,5 mM Glucose, 23,6 Tris Base, 1,3 mM $CaCl_2$, 0,03 mM EDTA-Na, 0,1 mM Ascorbinsäure, 0,01 mM Nialamid.

Die Reaktion verläuft in 0,1 ml Inkubationslösung in Glasepruvetten. Jeder Probe werden 0.7 ml des Puffers, 0,1 ml der untersuchten Verbindungen in verschiedenen Konzentrationen (oder 0.1 ml des Puffers und TWIN-80 für die Blind-und Kontrollprobe), 0,1 ml der synaptosomen Suspension der Gehirnrinde (5-HT), Hypothalamus (NA), Corpus stritum (DA) zugefügt. Jeder Versuch besteht aus einer Blindprobe (die Inkubation erfolgt im Eisbad), einer Kontrollprobe (ohne Inhibitoren des uptake) und einer Versuchsprobe aus den zu untersuchenden Verbindungen. Nach 10 minütiger Inkubation bei 37 °C beginnt die Reaktion nach Zugabe von 0,1 ml angesäuerter NaCl-Lösung (0.15 M NaCl und 0,01 n HCl), enthaltend 100-300 mM (-)-$)^3H$(-NA, 200-250 nM) $^3H$ (-5-HT und 50-300 nM) $^3H$ (-DA). Die Proben inkubiert man 6 min für 5-HT, 10 min für NA und 3 min für DA bei 37 °C. Die Reaktion wird nach Zugabe von 4 ml eiskalter Lösung aus 0,9% NaCl unterbrochen. Das synaptosome Material wird von dem Inkubationsgemisch auf Milliporfilter getrennt (0,45 μ) und einmal mit 4 ml eiskalter 0,9%iger NaCl-Lösung gewaschen. Jeder Filter wird in ein Scintillationsgefäß gebracht unter Verwendung einer Standard-Scintillationslösung. Die Radioaktivität wird am flüssigen Scintillationszähler gemessen.

Beispiel 6

Bestimmung der inhibierenden Wirkung von 1a und 1b auf durch Wasser-Immersionsstreß verursachte Magengeschwüre bei Ratten

Die Versuche wurden mit 280 weißen männlichen Ratten der Rasse Wistar mit einem Gewicht von 150 bis 200 g nach der in K. Takadi, Y. Ischii, Arzneim. Forsch. Drug Res., 1967, 17, 1544, beschriebenen Methode durchgeführt. 18 h vor dem Versuch hungern die Tiere unter freiem Wasserzutritt. Die Versuchsverbindung wird oral verabreicht, worauf die Tiere unbeweglich und voneinander isoliert auf dem Rücken auf eine Unterlage gebunden werden. Man legt die Tiere bei 17-18 °C ins Wasser und tötet sie nach 5 h. Die Mägen werden entnommen und die destruktiven Veränderungen makroskopisch bestimmt.

INHIBIERENDE WIRKUNG VON 4-(4′-HALOPHENYL)-2-METHYL-1,2,3,4-TETRAHYDROISOCHINOLIN AUF UPTAKE VON DOPAMIN (DA), NORADRENALIN (NA) UND SEROTONIN (5-HT)

Tabelle 1

| Verbindung | R | DA | NA | 5-HT |
|---|---|---|---|---|
| | | Aktivität (M) | | |
| 1a | Br | $10^{-7}$ | $10^{-8}$ | $10^{-7}$ |
| 1b | F | $10^{-7}$ | $10^{-8}$ | $10^{-7}$ |
| Nomifensin | - | $10^{-7}$ | $10^{-8}$ | $10^{-5}$ |

EINFLUSS VON 4-(4'-HALOPHENYL)-2-METHYL-1,2,3,4-TETRAHYDROISOCHINOLIN AUF EIN VERSUCHSMODELL VON DURCH WASSER-IMMERSIONSSTRESS VERURSACHTEN MAGENGESCHWÜREN UNTER ORALER VERABREICHUNG

Tabelle 2

| 1a (R = Br) | | | | |
|---|---|---|---|---|
| Verbindung | Dosis mg/kg | Anzahl der Tiere | Geschwürindex | Unterdrückung des Geschwürindex 4 |
| 1a | Kontrolle | 15 | 74,26±29,64 | - |
| R = Br | 0,050 | 12 | 69 ±18,2 | 20,6 |
| | 0,100 | 12 | 44,4 ± 9,66 | 48,3 |
| | 0,500 | 10 | 30,8 ±17,7 | 52,1 |
| | 1,000 | 11 | 35,7 ±14,2 | 58,9 |
| | 3,000 | 10 | 19,5 ±12,3 | 70,3 |
| 1b | Kontrolle | 12 | 65,83±38,19 | - |
| R = F | 1,000 | 8 | 38,5 ± 7,75 | 41,53 |
| | 3,000 | 9 | 27,66±12,73 | 57,98 |
| Ranitidin | Kontrolle | 10 | 44,3 | - |
| | 0,500 | 10 | 32,3 | 27 |
| | 1,000 | 10 | 26,9 | 39,27 |
| | 3,000 | 10 | 28,4 | 35,89 |
| | 10 | 10 | 22,8 | 46,98 |
| | 30 | 10 | 17,0 | 61.62 |

## Ansprüche

1. 4-(4′-Halophenyl)-2-methyl-1.2,3,4-tetrahydroisochinolin, Razemate oder optisch aktive Isomere der Formel 1

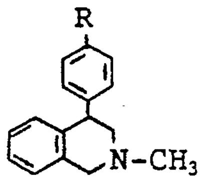

(1)

wobei R Brom oder Fluor bedeutet und ihre physiologisch verträgliche Salze.

2. Verfahren zur Herstellung von razemischen Tetrahydroisochinolinen der Formel 1 nach Anspruch 1, **dadurch gekennzeichnet,** daß Benzylmethylamin mit α-halogenierten arylaliphatischen Ketonen zu 2-(N-Methyl-N-benzalamino)-4′-haloacetophenonen alkyliert wird, die mit Natriumborhydrid bis zu den N-Benzyl-2-methylamin-1-(4′halophenyl)-1-ethanolen reduziert werden, wonach unter dem Einfluß von konz. Schwefelsäure letztere cyclodehydratisiert werden.

3. Verfahren zur Herstellung optisch aktiver Tetrahydroisochinoline der Formel 1 gemäß Anspruch 1, **dadurch gekennzeichnet,** daß die razemischen Tetrahydroisochinoline mit optisch aktiven Säuren behandelt werden.

4. Verfahren nach Anspruch 2, **dadurch gekennzeichnet,** daß die Alkylierung des Benzylmethylamins unter doppeltem molarem Überschuß des letzteren in Aceton oder Benzol erfolgt.

5. Verfahren nach Anspruch 2. **dadurch gekennzeichnet,** daß die Hydrierung der Ketone in niedrigen Alkoholen im Temperaturbereich von 10 bis 80 °C erfolgt.

6. Verfahren nach Anspruch 2. **dadurch gekennzeichnet,** daß die Cyclodehydratisierung der Alkohole in Dichlormethan oder Dichlorethan im Temperaturbereich von 0 bis 30 °C erfolgt.

Europäisches Patentamt

# EUROPÄISCHER RECHERCHENBERICHT

Nummer der Anmeldung

EP 90 10 7538

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | JOURNAL OF HETEROCYCLIC CHEMISTRY Band 7, Nr. 1, 1970, Seiten 159-169; K. FRETER et al.: "A New Tetrahydroisoquinoline Synthesis" * Tabelle I, Verbindung 2b * --- | 1 | C 07 D 217/14<br>A 61 K 31/47 |
| A | JOURNAL OF MEDICINAL CHEMISTRY Band 30, Nr. 10, 1987, Seiten 1887-1891; R.M. RIGGS et al.: "Evaluation of Isomeric 4-(Chlorohydroxyphenyl)-1,2,3,4-tetrahydroisoquinolines as Dopamine D-1 Antagonists" * Seiten 1888, Verbindung 5 * --- | 1 | |
| A | JOURNAL OF HETEROCYCLIC CHEMISTRY Band 17, November 1980, Seiten 1563-1568; G. BOBOWSKI et al.: "Preparation of 4-Aryl-1,2,3,4-tetrahydro-3,3-dimethylisoquinolines and 1,2,3,4-Tetrahydro-3,3-dimethyl-4-phenylbenz(h)isoquinolines" * Seite 1564, Schema I; Tabelle II, Verbindung 3f * --- | 1,2 | RECHERCHIERTE SACHGEBIETE (Int. Cl.5) |
| P,X | FARMACIJA Band 39, Nr. 6, 1989, Seiten 4-8; I. DOICHINOVA et al.: "Acid-base Behavior of a Series of 4-Phenyl-1,2,4,5-tetrahydroisoquinolines - Experimental Determination and Theoretical Calculation" * Seite 5, Tabelle 1, Verbindungen AN12,AN13 * ----- | 1 | C 07 D 217/00<br>A 61 K 31/00 |

Der vorliegende Recherchenbericht wurde für alle Patentansprüche erstellt

| Recherchenort | Abschlußdatum der Recherche | Prüfer |
|---|---|---|
| BERLIN | 27-08-1990 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)